Europäisches Patentamt

European Patent Office

Office européen des brevets

(19)

(11) Veröffentlichungsnummer: **0 178 581 B1**

(12) # EUROPÄISCHE PATENTSCHRIFT

(45) Veröffentlichungstag der Patentschrift: **22.01.92**

(21) Anmeldenummer: **85112794.4**

(22) Anmeldetag: **09.10.85**

(51) Int. Cl.⁵: **A61K 31/415, A23K 1/16**

(54) **Verwendung von 2-[(2-Brom-6-fluorphenyl)imino]imidazolidin als Sedativum.**

(30) Priorität: **15.10.84 DE 3437694**

(43) Veröffentlichungstag der Anmeldung:
**23.04.86 Patentblatt 86/17**

(45) Bekanntmachung des Hinweises auf die
Patenterteilung:
**22.01.92 Patentblatt 92/04**

(84) Benannte Vertragsstaaten:
**AT BE CH DE FR GB IT LI LU NL SE**

(56) Entgegenhaltungen:
**EP-A- 0 170 193**
**DE-A- 2 630 060**

**PROGRESS IN PHARMACOLOGY, Band 3, Nr. 1, 1980, Seiten 1-104, Gustav Fischer Verlag, Suttgart, DE; P.B.M.W.M. TIMMERMANS et al.: "Structure-activity relationships in clonidine-like imidazolidines and related compounds"**

(73) Patentinhaber: **BOEHRINGER INGELHEIM VET-MEDICA GMBH**

**W-6507 Ingelheim/Rhein(DE)**

(72) Erfinder: **Kern, Otto, Dr.**
**Rinderbachstrasse 33**
**W-6507 Ingelheim am Rhein(DE)**
Erfinder: **Wilhelm, Franz, Dr.**
**Theodor-Fliedner-Strasse 12**
**W-6507 Ingelheim am Rhein(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung einer Substanz mit sedierender, analgetischer und bradycarder Wirkung.

In der DE-PS 2 630 060 ist die Synthese von 2-[(2-Brom-6-fluorphenyl)imino]imidazolidin(I) offenbart, wobei als pharmakologische Eigenschaft dieser Substanz eine blutdrucksenkende Wirkung beschrieben wird. In"Structure Activity Relationships in Clonidine-like Imidazolidines and Related Compounds" sind die pharmakologischen Eigenschaften von Imidazolin-Derivaten offenbart, wobei für das 2-[(2-Brom-6-fluorphenyl)imino]imidazolidin lediglich die blutdrucksenkenden und antisecretorischen Eigenschaften besonders hervorgehoben sind.

Bei der Suche nach einer Substanz, die als Sedativum für Schweine Verwendung finden könnte, fiel unerwartet die in der DE-PS 26 30 060 beschriebene Verbindung sowie deren Additionssalze als besonders wirksam auf. Überraschend war, daß offenbar tierartspezifisch die sedierende Wirkung im Vordergrund steht und eine mögliche Blutdrucksenkung nur als unerhebliche Nebenwirkung, nicht aber, wie eigentlich erwartet, als Hauptwirkung beobachtet wurde.

Bei weiteren Versuchen hat sich die Verbindung auch bei anderen Warmblütern wie z.B. Wiederkäuern, Pferden und Geflügel als wirksam erwiesen.

Zusätzlich zur sedierenden Eigenschaft konnte bei diesen Versuchen auch eine analgetische Wirkung gezeigt werden.

Die Verbindung kann auch zur Verstärkung und Verlängerung der Wirkung anderer Substanzen, wie z.B. Hypnotika und Narkotika eingesetzt werden.

Von besonderem Vorteil erweist sich die Tatsache, daß 2[(2-Brom-6-fluorphenyl)imino] imidazolidin und dessen Additionssalze eine ausgeprägte bradycarde Wirkung aufweist, wobei außer durch die allgemeine Sedierung auch eine direkte Schonung des Herzens bei Aufregungszuständen erreicht wird, wie es bei den üblichen Neuroleptika der Phenothiazin- oder Haloperidol-Reihe nicht gegeben ist.

Die Überlegenheit von 2-[(2-Brom-6-fluorphenyl)imino]imidazolidin oder dessen Säureadditionssalze bei der Verwendung als Sedativum mit analgetischer und bradycardisierender Wirkung auch gegenüber der konstitutionell ähnlichsten Verbindung, dem Dichlorphenyl-Derivat, zeigt ein Vergleich der analgetischen und bradycardisierenden Wirkungen in Tab. I.

| Tab. I | Writhing Test ($ED_{50}$) | Bradicardisierende Wirkung ($ED_{60}$) |
|---|---|---|
| 2-[(2-Brom-6-fluorphenyl)-imino]imidazolidin  (I) ·HCL | 0.004 mg/kg | 0.003 mg/kg |
| 2-[(2,6-Dichlorphenyl)imino]-imidazolidin · HCL | 0.017 mg/kg | 0.010 mg/kg |

Das vorteilhafte Zusammenwirken der sedierenden, analgetischen und bradycarden Eigenschaften dieser Verbindung eröffnet ein breites Spektrum zur Verwendung dieser Substanz in der Veterinärmedizin, so z.B.
- zur Behandlung von Streßzuständen, ausgelöst z.B. durch zu dichte Belegung der Schlaf- und Futterplätze, Rangkämpfe, Transporte und Geburt.
- Zur Verhinderung der Foetophagie bei Muttersauen.
- Zur Verwendung bei der Neuroleptanalgesie und Anästhesie.
- Zur Sedation und Imobilisation von Wildtieren.

Die Erfindung betrifft die Verwendung einer Substanz mit sedierender, analgetischer und bradycarder Wirkung.

Die wirksamen Dosierungen liegen im Bereich von 0.002 bis 5.0 mg/kg Körpergewicht, bevorzugt im Bereich von 0.006 bis 0.8 mg/kg Körpergewicht. Als besonderer Vorteil ist anzusehen, daß 2-[(2-Brom-6-fluorphenyl)imino]imidazolidin nicht nur nach Injektion, sondern auch nach oraler Verabreichung in diesem Dosisbereich wirksam ist.

Unabhängig von der Applikationsart ist der Effekt dosisabhängig in Stärke und Dauer steigerbar (entsprechende Untersuchungen sind in den Beispielen aufgeführt).

Die in Frage kommenden Darreichungsformen sind übliche Zubereitungen für die parenterale, orale, rektale oder dermale Applikation.

Für eine parenterale Injektion ist eine 0.1 bis 1%-ige wässrige Lösung des 2[(2-Brom-6-fluorphenyl)-imino]imidazolidinhydrochlorids bevorzugt.

Orale Zubereitungen (z.B. Lösung, Pulver, Tabletten, Kapseln) können eingegeben oder mit dem Trinkwasser oder Futter verabreicht werden.

Die folgenden Beispiele beschreiben Dosis-Wirkungsbeziehungen von 2-[(2-Brom-6-fluorphenyl)imino]-imidazolidinhydrochlorid.

## Beispiele

| Tierart | Dosis mg/kg | Verabreichung | Wirkung |
|---------|-------------|---------------|---------|
| Pferd | 0,03 | i.v. | leichte Sedation, geringe Analgesie |
| | 0,03 | i.m. | leichte Sedation, keine Analgesie |
| | 0,06 | i.v. | starke Sedation, gute Analgesie |
| | 0,06 | i.m. | mittelgradige Sedation, etwas schwächere Analgesie |
| | 0,07 | i.v. | starke Sedation, gute Analgesie |
| | 0,08 | i.m. | starke Sedation und gute Analgesie |
| Rind (Kalb) | 0,006 | i.v. i.m. | geringgradige Sedation, keine Analgesie |
| | 0,008 | i.v. | gute Sedation, tiefer Schlaf |
| | 0,009 | i.v. | sehr gute Sedation, tiefer Schlaf, Analgesie: nicht vollständig |
| | 0,009 | i.m. | sehr gute Sedation, tiefer Schlaf, Analgesie: analog wie nach i.v. |
| | 0,01 | i.v. | sehr gute Sedation, mittelgradige Analgesie |
| | 0,01 | i.m. | sehr gute Sedation, mittelgradige Analgesie |
| Kalb | 0,01 | i.v. | als Prämedikation für chirurgische Eingriffe – Hernia umbilicalis – 20 Minuten post injectionem wurde die Nabelbruchoperation durchgeführt, die Sedation und Analgesie während und nach der Operation waren als gut zu bewerten. |
| | 0,4 | p.o. | sehr gute Sedationstiefe, Tier nicht auftreibbar. |

| Tierart | Dosis mg/kg | Verabreichung | Wirkung |
|---|---|---|---|
| Schwein | 0,01-0,03 | i.m. | schwache Sedation und Analgesie |
| | 0,03-0,06 | i.m. | gute Sedation, etwas schwächere Analgesie |
| | 0,06-0,1 | i.m. | sehr gute Sedation und gute Analgesie |
| | 0,1-1,0 | i.m. | starke Sedation und sehr gute Analgesie |
| Geflügel | 0,01-0,1 | i.m. | schwache Sedation |
| | 0,1-0,5 | i.m. | gute Sedation |
| | 0,5-1,0 | i.m. | sehr gute Sedation |
| | 0,5-1,0 | p.o. | gute Sedation |
| | 2,0 | i.m. | starke Sedation |
| | 2,0 | p.o. | gute Sedation |

Herstellungsbeispiel:

2-[(2-Brom-6-fluorphenyl)imino]imidazolidinhydrochlorid

a) Ausgangsprodukt

Durch Umsetzung von 2-Fluoranilin mit Acetanhydrid erhält man 2-Fluoracetanilid; Fp.: 77 bis 78,5°C, welches durch Reaktion mit Chlorsulfonsäure in 4-Chlorsulfonyl-2-fluoracetanilid, Fp. 110 bis 13o°C (Rohprodukt) überführt wird. Umsetzung mit konzentriertem Ammoniak ergibt das entsprechende Sulfonamid, Fp. 162 bis 165°C, das bromiert und dann mit konzentrierter Säure zum 2-Brom-6-fluoranilin desulfonyliert und gleichzeitig verseift wird. Das rohe, ölige Anilin wird ohne Reinigung mit Kaliumthiocyanat/Benzylchlorid und anschließende Verseifung mit Kalilauge in N-(2-Brom-6-fluorphenyl)-thioharnstoff, Fp. 145 bis 147°C überführt, aus dem man durch Methylierung mit Methyljodid N-(2-Brom-6-fluorphenyl)-S-methyl-isothiuroniumjodid gewinnt.

b) Endprodukt

6,7 g (0,017 Mol) N-(2-Brom-6-fluorphenyl)-S-methyl-isothiuroniumjodid werden zusammen mit 1,7 ml (150 %) Äthylendiamin in 2o ml n-Butanol 6 Stunden lang unter Rühren am Rückfluß erhitzt. Hierauf wird im Vakuum zur Trockne eingeengt und der verbleibende Rückstand in verdünnter Salzsäure gelöst. Es wird 2 mal ausgeäthert (Ätherextrakte werden verworfen) und anschließend bei aufsteigenden pH-Werten (Alkalisieren mit verdünnter Natronlauge) fraktioniert mit Äther extrahiert. Die dünnschichtchromatographisch reinen Fraktionen werden vereinigt, über Magnesiumsulfat getrocknet und über Aktivkohle filtriert. Durch Zugabe von ätherischer Salzsäure bis zur kongosauren Reaktion wird das Imidazolidinhydrochlorid gefällt. Nach Absaugen, Waschen mit absolutem Äther und Trocknen erhält man eine Ausbeute von 2,7 g entsprechend 53,6 % der Theorie der Titelverbindung vom Schmelzpunkt 259 bis 262°C. Der pKa-Wert der Substanz beträgt 8,2.

$C_9H_9BrFN_3$ x HCl, Mol-Gew.: 294,54.

Formulierungsbeispiele

Tabletten
----------

| | |
|---|---:|
| Wirkstoff | o,15 mg |
| Maisstärke | 160,oo mg |
| sek. Calciumphosphat | 25o,oo mg |
| Magnesiumstearat | 9,85 mg |
| insgesamt | 42o,oo mg |

Herstellung:

Die einzelnen Bestandteile werden intensiv miteinander vermischt und die Mischung in üblicher Weise granuliert. Das Granulat wird zu Tabletten von 42o mg Gewicht verpreßt, von denen jede o,15 mg Wirkstoff enthält.

Gelatinekapseln
---------------

Der Inhalt der Kapseln setzt sich wie folgt zusammen:

| | |
|---|---:|
| Wirkstoff | o,3 mg |
| Maisstärke | 199,7 mg |
| | 2oo,o mg |

Herstellung:

Die Bestandteile des Kapselinhalts werden intensiv vermischt und 2oo mg Portionen der Mischung in Gelatinekapseln geeigneter Größe abgefüllt. Jede Kapsel enthält o,3mg des Wirkstoffs.

Injektionslösung
----------------

Die Lösung wird aus folgenden Bestandteilen hergestellt:

| | | |
|---|---|---:|
| Wirkstoff | | 1,5 Teile |
| Natriumsalz der Äthylendiamin-tetraessigsäure | | o,2 Teile |
| dest. Wasser | ad | loo,o Teile |

Herstellung:

Der Wirkstoff und das Natriumsalz der Äthylendiamin-tetraessigsäure werden in genügend Wasser gelöst und mit Wasser auf das gewünschte Volumen aufgefüllt. Die Lösung wird von suspendierten Partikeln filtriert und in 2 ml Ampullen unter aseptischen Bedingungen abgefüllt. Zuletzt werden die Ampullen sterilisiert und verschlossen.
Jede Ampulle enthält 3o mg Wirkstoff.

## Patentansprüche

1. Verwendung von 2-[(2-Brom-6-fluorphenyl)imino]imidazolidin oder dessen verträglichen Additionssalzen zur Herstellung eines Arzneimittels mit sedierender, analgetischer und bradycarder Wirkung im Rahmen veterinärmedizinischer oder tierschützerischer Maßnahmen.

2. Verwendung von 2-[(2-Brom-6-fluorphenyl)imino]imidazolidinhydrochlorid nach Anspruch 1.

3. Verwendung von 2-[(2-Brom-6-fluorphenyl)imino]imidazolidin oder dessen verträglichen Additionssalzen zur Herstellung eines Arzneimittels mit sedierender, analgetischer und bradycarder Wirkung zur Behandlung von Streßzuständen.

4. Verwendung von 2-[(2-Brom-6-fluorphenyl)imino]imidazolidin oder dessen verträglichen Additionssalzen zur Herstellung eines Arzneimittels mit sedierender, analgetischer und bradycarder Wirkung zur Verhinderung der Foetophagie.

5. Verwendung von 2-[(2-Brom-6-fluorphenyl)imino]imidazolidin oder dessen verträglichen Additionssalzen zur Herstellung eines Arzneimittels mit sedierender, analgetischer und bradycarder Wirkung zur Verwendung bei der Neuroleptanalgesie.

6. Verwendung von 2-[(2-Brom-6-fluorphenyl)imino]imidazolidin oder dessen verträglichen Additionssalzen zur Herstellung eines Arzneimittels mit sedierender, analgetischer und bradycarder Wirkung zur Sedation und Imobilisation von Wildtieren.

7. Verwendung von 2-[(2-Brom-6-fluorphenyl)imino]imidazolidin oder dessen verträglichen Additionssalzen zur Herstellung eines Arzneimittels zur Sedation, Analgesie und Bradycardie, wobei das Arzneimittel dem Futter oder Trinkwasser beigegeben ist.

## Claims

1. Use of 2-[(2-bromo-6-fluorophenyl)imino]imidazolidine or the acceptable acid addition salts thereof for preparing a pharmaceutical composition with sedative, analgesic and bradycardiac effects, in veterinary medicine or animal conservation procedures.

2. Use of 2-[(2-bromo-6-fluorophenyl)imino]imidazolidine hydrochloride as claimed in claim 1.

3. Use of 2-[(2-bromo-6-fluorophenyl)imino]imidazolidine or the acceptable addition salts thereof for preparing a pharmaceutical composition with sedative, analgesic and bradycardiac effects for treating stress.

4. Use of 2-[(2-bromo-6-fluorophenyl)imino]imidazolidine or the acceptable addition salts thereof for preparing a pharmaceutical composition with sedative, analgesic and bradycardiac effects for preventing the eating of young.

5. Use of 2-[(2-bromo-6-fluorophenyl)imino]imidazolidine or the acceptable addition salts thereof for preparing a pharmaceutical composition with sedative, analgesic and bradycardiac effects for use in neuroleptanalgesia.

6. Use of 2-[(2-bromo-6-fluorophenyl)imino]imidazolidine or the acceptable addition salts thereof for preparing a pharmaceutical composition with sedative, analgesic and bradycardiac effects for the sedation and immobilisation of wild animals.

7. Use of 2-[(2-bromo-6-fluorophenyl)imino]imidazolidine or the acceptable addition salts thereof for preparing a pharmaceutical composition for sedation, analgesia and bradycardia, the composition being added to the fodder or drinking water.

## Revendications

1. Utilisation de la 2-[(2-bromo-6-fluorophényl)imino]imidazolidine ou de ses sels d'addition compatibles pour la préparation d'un médicament à action sédative, analgésique et bradycardisante dans le cadre de mesures de médecine vétérinaire ou de protection des animaux.

2. Utilisation du chlorhydrate de la 2-[(2-bromo-6-fluorophényl)imino]imidazolidine selon la revendication 1.

3. Utilisation de la 2-[(2-bromo-6-fluorophényl)imino]imidazolidine ou de ses sels d'addition compatibles pour la préparation d'un médicament a action sédative, analgésique et bradycardisante pour le traitement d'états de stress.

4. Utilisation de la 2-[(2-bromo-6-fluorophényl)imino]imidazolidine ou de ses sels d'addition compatibles pour la préparation d'un médicament à action sédative, analgésique et bradycardisante pour éviter la foetophagie.

5. Utilisation de la 2-[(2-bromo-6-fluorophényl)imino]imidazolidine ou de ses sels d'addition compatibles pour la préparation d'un médicament à action sédative, analgésique et bradycardisante en vue d'une utilisation pour la neuroleptanalgésie.

6. Utilisation de la 2-[(2-bromo-6-fluorophényl)imino]imidazolidine ou de ses sels d'addition compatibles pour la préparation d'un médicament à action sédative, analgésique et bradycardisante pour la sédation et l'immobilisation d'animaux sauvages.

7. Utilisation de la 2-[(2-bromo-6-fluorophényl)imino]imidazolidine ou de ses sels d'addition compatibles pour la préparation d'un médicament pour la sédation, l'analgésie et la bradycardie, le médicament étant ajouté à la nourriture ou à l'eau de boisson.